# EUROPÄISCHE PATENTSCHRIFT

(11) **EP 2 173 421 B1**
(45) Veröffentlichungstag und Bekanntmachung des Hinweises auf die Patenterteilung: **10.04.2019**
(21) Anmeldenummer: 08786700.8
(22) Anmeldetag: 31.07.2008
(51) Int. Cl.: A61M 15/00

(54) **INHALATOR**
INHALATOR
INHALATEUR

(30) Priorität: 01.08.2007 EP 07113624
(43) Veröffentlichungstag der Anmeldung: 14.04.2010
(73) Patentinhaber: BOEHRINGER INGELHEIM INTERNATIONAL GMBH, 55216 Ingelheim am Rhein (DE); Vectura Delivery Devices Limited, Chippenham, Wiltshire SN14 6FH (GB)
(72) Erfinder: WACHTEL, Herbert, 55216 Ingelheim am Rhein (DE); HARMER, Quentin, Cambridge, Cambridgeshire CB4 0GZ (GB); MILIVOJEVIC, Ivan, Cambridge, Cambridgeshire CB4 0GZ (GB); SARKAR, Matthew, Cambridge, Cambridgeshire CB4 0GZ (GB)
(74) Vertreter: Summersell, Richard John
(86) Internationale Anmeldenummer: PCT/EP2008/060078
(87) Internationale Veröffentlichungsnummer: WO 2009/016238

(56) Entgegenhaltungen:
- EP-A- 0 041 783
- EP-A- 1 129 705
- EP-A- 1 760 008
- EP-A1- 2 082 764
- EP-A1- 2 082 769
- WO-A-2006/026237
- WO-A-2006/108877
- DE-A1- 10 239 443
- FR-A- 2 224 175
- US-A- 3 807 400
- US-A1- 2005 161 041

## Beschreibung

Die Erfindung bezieht sich auf einen Inhalator zur Verabreichung eines pulverförmigen Arzneimittels in Form einer inhalationsfähigen Substanz, Substanzformulierung oder - mischung aus einer mit Anstechelementen zu öffnenden Blisterkavität, die in einem Gehäuseunterteil eines Gehäuses gelagert ist, das aus einem als Mundstück ausgebildeten Gehäuseoberteil mit einem Inhalationskanal und dem eine Lufteintrittsöffnung aufweisenden Gehäuseunterteil zusammengesetzt ist.

Die EP 0 911 047 A1 offenbart einen Inhalator für die Inhalation pulverförmiger Arzneimittel aus Kapseln, der ein Unterteil mit zwei Fenstern und eine Platte, in der sich Kapselhalterungen sowie Lufteinlassöffnungen befinden, umfasst. Im Weiteren ist eine Inhalationskammer mit der Platte verbunden, an der ein mit zwei geschliffenen Nadeln versehener, gegen eine Feder beweglicher Kopf vorgesehen ist. Ein Mundrohr ist mit einem Oberteil des Inhalators und ein Deckel klappbar mit dem Unterteil, der Platte sowie dem Oberteil verbunden. Dieser Inhalator weist einen aufwändigen Aufbau auf und ist für eine Mehrfach-Verwendung vorgesehen.

Im Weiteren beschreibt die EP 0 835 148 B1 einen Inhalator zur Verabreichung von Arzneimitteln aus einer streifenförmigen Blisterpackung, wobei eine Blisterkavität mittels einer Andrückhilfe entleert wird. Der Inhalator besteht im Wesentlichen aus einem länglichen Gehäuse, das aus mindestens zwei über ein Scharnier schwenkbar miteinander verbundenen Gehäuseteilen besteht. In einem der Gehäuseteile ist eine Aussparung als Lager zur Aufnahme des Blisterstreifens ausgebildet. Das Gehäuse weist an einer Schmalseite ein Mundstück und auf der dem Mundstück gegenüberliegenden Schmalseite eine Lufteintrittsöffnung auf, die über einen Luftkanal mit dem Mundstück verbunden ist. Der Luftkanal ist zur Aufnahme des Medikaments aus der Blisterkavität ausgeführt, das mit einem dem Gehäuse zugeordneten Ausdrückstempel freigesetzt wird, wobei der Ausdrückstempel bei einer Druckbetätigung durch einen Benutzer des Inhalators ein Aufreißen einer Deckelfolie der Blisterkavität bewirkt, worauf das Arzneimittel entweder in der Kavität des Blisters hängen bleibt oder in einen Pulverkanal des Luftkanals fällt. Dieser Inhalator ist aufgrund seines Aufbaus zum mehrmaleigen Gebrauch bestimmt.

Um das Arzneimittel wirkungsvoll zu inhalieren, muss der Patient das Mundstück des Inhalators mit den Mundschleimhäuten (Lippen, Mund/Rachenraum) in Verbindung bringen. Dies erweist sich insofern als problematisch, als die Mundschleimhäute bei allen Menschen eine unterschiedlich große Anzahl verschiedenster Bakterien sowie andere Mikroorganismen aufweisen, die gegebenenfalls Krankheitserreger sein können. Demnach wird das Mundstück des Inhalators beim Gebrauch kontaminiert. Die Patienten und damit die Benutzer von Inhalatoren sind zur Reinigung des Mundstücks nach Gebrauch des Inhalators angehalten, wobei der Reinigungsprozess allerdings in Abhängigkeit von der persönlichen Vorgehensweise der Patienten, ihrem Alter und ihrem Erkrankungsgrad unterschiedlich konsequent durchgeführt wird. Darüber hinaus ist auch das Innere des Gehäuses des Inhalators, insbesondere von Arzneimittelrückständen, zu reinigen, da diese Rückstände zu regulatorischen Problemen führen können, wenn sie sich in unregelmäßigen Abständen lösen und mit der eigentlichen Dosis ausgebracht werden.

Die DE 693 19 100 T2 zeigt einen durch Atmung zu aktivierenden Wegwerfinhalator, der ein röhrenförmiges Gehäuse mit zwei einen an beiden Enden offenen Luftströmungsweg bildenden Teilen umfasst, wobei ein Ende einen Lufteinlass und das andere Ende einen Luftauslass bildet. Das Gehäuse weist ein Kompartiment zur Aufbewahrung eines pharmazeutischen Pulvers zur Inhalation auf und ist mit einer Verengung neben dem Kompartiment ausgebildet, um bei einer Inhalation eine turbulente Luftströmung an der Verengung zu erzielen, durch die das Pulver aus dem Kompartiment angehoben und mit dem Luftstrom vermischt wird. Das Kompartiment ist als Vertiefung neben dem Lufteinlass ausgebildet, die über ein Durchgangsloch in ihrem Boden mit der Umgebungsluft in Verbindung steht. Die Vertiefung und das Durchgangsloch sind mit einer abdichtenden Folie luftdicht abgedeckt, wobei die Folie von der Außenseite her abgelöst werden kann.

Es ist Aufgabe der Erfindung, einen Inhalator der eingangs genannten Art zu schaffen, der bei einem einfachen und kostengünstigen Aufbau für einen Patienten einfach handhabbar ist.

Erfindungsgemäß wird die Aufgabe dadurch gelöst, dass der Inhalationskanal des Gehäuseoberteils eine Einheit zum Dispergieren des pulverförmigen Arzneimittels aufweist, die mit den Anstechelementen verbunden ist, wobei das Gehäuseoberteil zum Öffnen der Blisterkavität relativ zu dem Gehäuseunterteil verlagerbar ist.

Der Inhalator ist insofern vorteilhaft, als er unter Verwendung einer geringen Anzahl von Einzelteilen kostengünstig zur einmaligen Verwendung herstellbar ist und lediglich absolut notwendige Bauteile umfasst. Die Einheit zum Dispergieren ist entweder im Spritzgussverfahren einteilig mit dem Gehäuseobersteil hergestellt oder mit dem Inhalationskanal durch Schweißen, Kleben, Pressen oder andere bekannte Verbindungstechniken fest und dauerhaft verbunden. Die Einheit zum Dispergieren sorgt für eine feine inhalierfähige Verteilung der Partikel des Arzneimittels in der von dem Benutzer angesaugten Luft. Durch die Ausgestaltung als Einmal-Inhalator ist dessen Handhabung vereinfacht, da eine regelmäßige Reinigung entfällt und Arzneimittelrückstände, insbesondere in dem Inhalationskanal und/oder der Einheit zum Dispergieren, die Abgabe des Arzneimittels nicht beeinträchtigen können. Das Gehäuseoberteil und damit auch das Mundstück kann ohne eine ergonomische Gestaltung als einfaches Rohr ausgebildet und ebenso wie das Gehäuseunterteil aus einem Kunststoff gefertigt sein. Vorzugsweise handelt es sich bei den Kunststoffen um Polymerisate, thermoplastische Polykondensate, Polyaddukte, abgewandelte Naturstoffe oder Kautschuke bzw. um Gemische dieser Kunststoffe. Besonders bevorzugt sind hier Polyolefine, Vinylchlorid-Polymerisate, Styrol-Polymerisate, Polyacetale, Polyamide, thermoplastische Polyester und Polyarylether bzw. Gemische dieser Kunststoffe. Beispiele für diese Kunststoffe sind Polyethylen, Polyvinylchlorid, Polyoxymethylen, Polyacetal, Acrylnitril/Butadien/Styrol(ABS), Acrylnitril/Styrol/Acryl-ester(ASA), Polyamide, Polycarbonat, Poly (ethylenterephthalat), Poly(butylenterephthalat) oder Poly(phenylenether). Derartige Kunststoffe können beispielsweise von der Firma Ensinger in Deutschland, Nufringen, bezogen werden.

Inhalatoren sind unter den Markennamen HandiHaler®, Spinhaler®, Rotahaler®, Aerolizer®, Flowcaps®, Turbospin®, AIR DPI®, Orbital®, Directhaler® bekannt und/oder in DE 33 45 722, EP 0 591 136, DE 43 18 455, WO 91/02558, FR-A-2 146 202, US-A-4 069 819, EP 666085, EP 869079, US 3,991,761, WO 99/45987, WO 200051672, Bell, J. Pharm. Sci. 60, 1559 (1971); Cox, Brit. Med. J. 2, 634 (1969), beschrieben. Als Pulverinhalatoren sind Einfach- oder Mehrfachdosis-Pulverinhalatoren, insbesondere der Spinhaler®, Rotahaler®, Aerolizer®, Inhalator®, HandiHaler®, Diskhaler®, Diskus®, Accuhaler®, Aerohaler®, Eclipse®, Turbohaler®, Turbuhaler®, Easyhaler®, Novolizer®, Clickhaler®, Pulvinal®, Novolizer®, SkyeHaler®, Xcelovair®, Pulvina®, Taifun®, MAGhaler®, Twisthaler® und der Jethaler® bekannt.

Die unten genannten Verbindungen können allein oder in Kombination zur Anwendung in der erfindungsgemäßen Vorrichtung gelangen. In den unten genannten Verbindungen ist W einen pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmern, Dopamin-Agonisten, H1-Antihistaminika, PAF-Antagonisten und PI3-Kinase Inhibitoren. Weiterhin können zwei- oder dreifach Kombinationen von W kombiniert werden und zur Anwendung in der erfindungsgemäßen Vorrichtung gelangen. Beispielhaft genannte Kombinationen von W wären:
- W stellt ein Betamimetika dar, kombiniert mit einem Anticholinergika, Corticosteroide, PDE4-Inhibitore, EGFR-Hemmern oder LTD4-Antagonisten,
- W stellt ein Anticholinergika dar, kombiniert mit einem Betamimetika, Corticosteroiden, PDE4-Inhibitoren, EGFR-Hemmern oder LTD4-Antagonisten,
- W stellt ein Corticosteroiden dar, kombiniert mit einem PDE4-Inhibitoren, EGFR-Hemmern oder LTD4-Antagonisten
- W stellt ein PDE4-Inhibitoren dar, kombiniert mit einem EGFR-Hemmern oder LTD4-Antagonisten
- W stellt ein EGFR-Hemmern dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HOKU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{[2-{[3-(2-phenylethoxy)propyl]sulphonyl} ethyl]-amino}ethyl]-2(3H)-benzothiazolon
- 1-(2-Fluor-4-hydroxyphenyl)-2-[4-(1-benzimidazolyl)-2-methyl-2-butylamino]ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl}-4H-benzo[1,4]oxazin-3-on
- 8-{2-[1,1-Dimethyl-2-(2,4,6-trimethylphenyl)-ethylamino]-1-hydroxy-ethyl}-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8-{1-hydroxy-2-[2-(4-isopropyl-phenyl)-1,1dimethyl-ethylamino]-ethyl}-4H-benzo[1,4]oxazin-3-on
- 8-{2-[2-(4-Ethyl-phenyl)-1,1-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl)-2-(tert.-butylamino)ethanol
- 2-Hydroxy-5-(1-hydroxy-2-{2-[4-(2-hydroxy-2-phenyl-ethylamino)-phenyl]-ethylamino}-ethyl)-benzaldehyd
- N-[2-Hydroxy-5-(1-hydroxy-2- {2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyt]-ethylamino}-ethyl)-1H-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1H-quinolin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxy-ethyl)-2-hydroxymethyl-phenol
- 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydroxy-2-(4-hydroxy-3-hydroxymethyl-phenyl)-ethylamino]-heptyloxy}-propyl)-benzylsulfonamid
- 4-(2-{6-[4-(3-Cyclopentanesulfonyl-phenyl)-butoxy]-hexylamino}-1-hydroxyethyl)-2-hydroxymethyl-phenol
- N-Adamantan-2-yl-2-(3-{2-[2-hydroxy-2-(4-hydroxy-3-hydroxymethyl-phenyl)-ethylamino]-propyl}-phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel **AC-1** 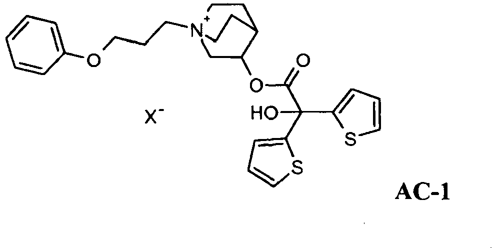 worin X⁻ ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel **AC-1-en** 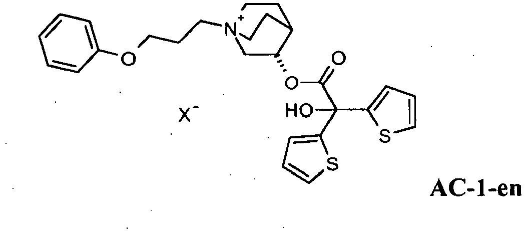 enthalten, worin X⁻ die vorstehend genannten Bedeutungen aufweisen kann. Weiterhin bevorzugte Anticholinergika sind ausgewählt aus den Salzen der Formel **AC-2** 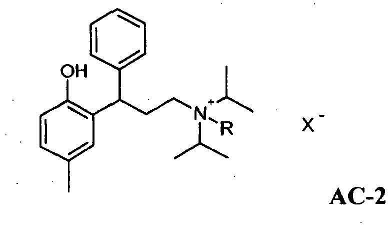 worin R entweder Methyl oder Ethyl bedeuten und worin X⁻ die vorstehend genannte

Bedeutungen aufweisen kann. In einer alternativen Ausführungsform kann die Verbindung der Formel **AC-2** auch in Form der freien Base **AC-2-base** vorliegen.

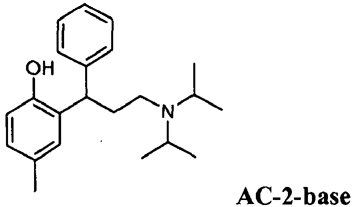

Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid

Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻ genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3S-yl)ester,
- 6α,9α-difluoro-11β-hydroxy-16α-methyl-3-oxo-17α-(2,2,3,3-tertamethylcyclo-propylcarbonyl)oxy-androsta-1,4-diene-17β-carbonsäure cyanomethyl ester gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, CI-1018, CDC-801, CDC-3052, D-22888, YM-58997, Z-15370 und
- N-(3,5-Dichloro-1-oxo-pyridin-4-yl)-4-difluormethoxy-3-cyclopropylmethoxybenzamid
- (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxy-phenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*-butyl)-9*H*-pyrazolo[3,4-c]-1,2,4-triazolo[4,3-a]pyridin gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der PDE4-Inhibitoren ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2-hydroxy-2-propyl)phenyl)thio)methylcyclopropan-essigsäure,
- 1-(((1(R)-3(3-(2-(2,3-Dichlorthieno[3,2-b]pyridin-5-yl)-(E)-ethenyl)phenyl)-3-(2-(1-hydroxy-1-methylethyl)phenyl)propyl)thio)methyl)cyclopropanessigsäure
- [2-[[2-(4-tert-Butyl-2-thiazolyl)-5-benzofuranyl]oxymethyl]phenyl]essigsäure gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chlor-4-fluorphenyl)amino]-6-{[4-(morpholin-4-yl)-1-oxo-2-buten-1-yl]-amino} -7-cyclopropylmethoxy-chinazolin
- 4-[(3-Chlor-4-fluorphenyl)amino]-6-{[4-(N,N-diethylamino)-1-oxo-2-buten-1-yl]-amino}-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethy))-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinyl-carbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{[4-(N,N-dimethylamino)-1-oxo-2-buten-1-yl]amino}-7-ethoxy-chinolin
- 4-{[3-Chlor-4-(3-fluor-benzyloxy)-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl]-amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxy-chinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yl-oxy}-7-methoxy-chinazolin
- 4-[(3-Chlor-4-fluor-phenyl)amino]-6-{1-[(methoxymethyl)carbonyl]-piperidin-4-yl-oxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin,
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6-{trans-4-[(dimethylamino)sulfonylamino]-cyclohexan-1-yloxy}-7-methoxy-chinazolin
- 4-[(3-Chlor-4-fluor-phenyl)amino]-6-{trans-4-[(morpholin-4-yl)carbonylamino]-cyclohexan-1-yloxy}-7-methoxy-chinazolin
- 4-[(3-Chlor-4-fluor-phenyl)amino]-6-{trans-4-[(morpholin-4-yl)sulfonylamino]-cyclohexan-1-yloxy}-7-methoxy-chinazolin
- 4-[(3-Chlor-4-fluor-phenyl)amino]-6-(tetrahydropyran-4-yloxy)-7-(2-acetylamino-ethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazolin
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy- chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin
- 4-[(3-Chlor-4-fluor-phenyl)amino]-6-(cis-4-(N-[(4-methyl-piperazin-1-yl)carbonyl]-N-methyl-amino)-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy} -7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6- {1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[(3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethy)-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy}-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethyl-amino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy} -7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-N-methyl-amino)-cyclohexan-1-yloxy]-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als pharmazeutisch wirksame Substanzen, Substanzformulierungen oder Substanzmischungen werden alle inhalierbaren Verbindungen eingesetzt, wie z.B. auch inhalierbare Makromoleküle, wie in EP 1 003 478 offenbart. Vorzugsweise werden Substanzen, Substanzformulierungen oder Substanzmischungen zur Behandlung von Atemwegserkrankungen eingesetzt, die im inhalativen Bereich Verwendung finden.

Weiterhin kann die Verbindung aus der Gruppe der Derivate von Mutterkornalkaloiden, der Triptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkornalkaloide: Dihydroergotamin, Ergotamin.

Bevorzugt sind die Anstechelemente in einer durch mindestens eine erste Clipsverbindung zwischen dem Gehäuseoberteil und dem Gehäuseunterteil arretierten Lage beabstandet zu der Blisterkavität ausgerichtet und ragen in einer durch mindestens eine zweite Clipsverbindung definierten Lage in die geöffnete Blisterkavität hinein. Aufgrund der ersten Clipsverbindung ist das Gehäuseoberteil zu dem Gehäuseunterteil in einer Lage derart sicher gehalten, dass ein unbeabsichtigtes Öffnen der Blisterkavität verhindert ist und aufgrund der zweiten Clipsverbindung ist eine einfache Handhabung des Inhalators beim Inhalieren sichergestellt, da das Gehäuseoberteil in der Lage, in der die Blisterkavität geöffnet ist und der Inhalator bestimmungsgemäß zum Inhalieren des Arzneimittels verwendet wird, fest an dem Gehäuseunterteil gehalten ist.

In Ausgestaltung weist jedes Anstechelement eine dreieckförmige Spitze auf, wobei ein Anstechelement im Bereich des Inhalationskanals und ein Anstechelement seitlich dazu versetzt angeordnet ist. Die im Wesentlichen messerspitzenförmigen Anstechelemente öffnen die Blisterkavität derart, dass beim Inhalieren durch die eine Öffnung ein Luftstrom in die Kavität eintritt und das pulverförmige Arzneimittel durch die andere Öffnung in den Inhalationskanal und von dort aus weiter zu dem Patienten mitnimmt. Aufgrund der Formgebung der Anstechelemente ist der Kraftaufwand seitens des Benutzers des Inhalators zum Einstechen der Deckfolie der Kavität des Blisters verhältnismäßig gering und die Ausbringungsrate des zu inhalierenden Arzneimittels sichergestellt.

Bei einer einmaligen Verwendung des Inhalators ist es nicht zwingend erforderlich, dass die Ansteckelemente eine lange Standzeit aufweisen, d. h. für die Öffnung einer Vielzahl von Blisterkavitäten geeignet sind. Daher sind zweckmäßigerweise die Anstechelemente aus einem Kunststoff gefertigt. Die Anstechelemente können beispielsweise aus einem Halbzeug hergestellt oder vorzugsweise einstückig mit der Einheit zum Dispergieren, beispielsweise im Spritzgussverfahren, gefertigt sein.

In einer alternativen Ausgestaltung bestehen die Anstechelemente aus Metall. Bei dem Metall kann es sich um einen so genannten Edelstahl handeln, der in der Medizintechnik Anwendung findet und eine gegenüber einem Kunststoff dünnere und spitzere Gestaltung der Anstechelemente ermöglicht, wodurch auch eine verhältnismäßig stabile die Kavität des Blisters überspannende Deckfolie einstechbar ist.

Vorzugsweise sind die Anstechelemente in einer Platte aus Metall freigestanzt und in einem spitzen Winkel derart zu der Platte abgewinkelt, dass sie in Richtung der Blisterkavität weisen. Die Platte mit den beiden Anstechelementen ist leicht handhabbar und montierbar. Im Weiteren gewährleistet die Geometrie der Anstechelemente im Zusammenwirken mit deren Stellung zur Deckfolie der Blisterkavität relativ große Strömungsöffnungen, durch die das Arzneimittel nahezu vollständig aus der Kavität ausgetragen wird. Zweckmäßigerweise ist die Platte form- und oder kraftschlüssig mit der Einheit zum Dispergieren verbunden. Die Platte kann beispielsweise als Einlegeteil ausgeführt und in einem Spritzgussvorgang mit der Einheit verbunden werden. Es ist aber auch möglich, die Platte mit zwei zueinander beabstandeten Bohrungen zu versehen, die von Kunststoffbolzen der Einheit zum Verschweißen durchragt werden. Selbstverständlich ist auch ein Verclipsen oder Verkleben der Platte mit der Einheit möglich.

Zweckmäßigerweise weist das Gehäuseunterteil zwei gegenüberliegende Clipnasen auf und in das Gehäuseoberteil sind dazu korrespondierende Clipsöffnungen in unterschiedlichen Ebenen eingelassen. Die Clipsöffnungen bestimmen in ihrem Zusammenwirken mit den Clipsnasen die Lage des Gehäuseunterteils zu dem Gehäuseoberteil sowohl im Auslieferungszustand des Inhalators, in dem die Blisterkavität ungeöffnet ist, als auch im Benutzungszustand, in dem die Blisterkavität durch die Anstechelemente weit geöffnet ist. Die Clipsöffnungen und die Clipsnasen sind fertigungstechnisch einfach und ohne zusätzlichen Kostenaufwand zu realisieren und der Benutzer hat eine taktil und optisch erfassbare Rückmeldung über die Lage des Gehäuseoberteils zu dem Gehäuseunterteil und damit auch über den Zustand des Inhalators. Im Weiteren lassen sich die Clipsverbindungen derart dimensionieren, dass zum einen ein unbeabsichtigten Verlagern des Gehäuseoberteils zu dem Gehäuseunterteil und ein damit verbundenes Öffnen der Blisterkavität verhindert und zum anderen ein Öffnen der Blisterkavität mit einem vertretbaren Kraftaufwand gegeben ist. Um die Betätigungskraft zum Verlagern des Gehäuseoberteils relativ zum Gehäuseunterteil zu reduzieren, sind die Clipsnasen an freien Enden von gegenüberliegenden Rastarmen angeordnet. Zur Erleichterung des Öffnens der Blisterkavität sind die Clipsöffnungen in der Ebene, in der die Blisterkavität geöffnet ist, langlochförmig gestaltet. In der Lage, in der die Clipsnasen die langlochförmigen Clipsöffnungen erreichen, dringen die Spitzen der Anstechelemente in die Deckfolie der Blisterkavität ein. Mit dem Zusammendrücken von Gehäuseunterteil und Gehäuseoberteil innerhalb des von den Langlöchern bestimmten Bereichs geht ein weiteres Einstechen und damit verbundenes Öffnen der Blisterkavität unter einem relativ geringen Kraftaufwand einher.

Bevorzugt ist das Gehäuseoberteil relativ zu dem Gehäuseunterteil verschiebbar gelagert. Die verschiebbare Lagerung ist einfach und unanfällig gegen Störungen zu realisieren. Eventuell kann eine Verdrehsicherung vorgesehen sein, die die Montage und Handhabung vereinfacht.

Nach einer Weiterbildung weist das Gehäuseoberteil einen zylindrischen oder elliptischen Querschnitt auf, in dem das im Querschnitt zylindrische oder elliptische Gehäuseunterteil gelagert ist und verläuft in Richtung einer Luftaustrittsöffnung konisch, wobei die Lufteintrittsöffnung an der freien Stirnseite des Gehäuseunterteils ausgebildet ist. Der konische Verlauf des zylindrischen bzw. elliptischen Gehäuseoberteils stellt eine dichte Anlage der Lippen des Benutzers des Inhalators an dem als Mundstück dienenden Gehäuseoberteil sicher, wodurch beim Inhalieren ein Hauptluftstrom durch den Inhalator eingesogen wird. Die Lufteintrittsöffnung an der freien Stirnseite des Gehäuseunterteils wirkt sich nicht störend auf die Handhabung des Inhalators beim Inhalieren aus, da das Gehäuse umfangsseitig festgehalten werden kann. Darüber hinaus sind die verhältnismäßig einfachen Geometrien des Gehäuseoberteils und des Gehäuseunterteils mit einem geringen Aufwand herzustellen.

Nach einer alternativen Weiterbildung ist das Gehäuseoberteil und das Gehäuseunterteil jeweils im Querschnitt wannenförmig ausgebildet, wobei das Gehäuseunterteil in dem Gehäuseoberteil gelagert ist. Demnach ist das aus dem Gehäuseoberteil und dem Gehäuseunterteil zusammengesetzte Gehäuse im Wesentlichen zylindrisch. Auch die einfach zu fertigende Wannenform stellt eine verhältnismäßig dichte Anlage der Lippen an dem Gehäuseunterteil sicher.

Um auf eine zusätzliche Verpackung des Arzneimittels zu verzichten, ist zweckmäßigerweise die Blisterkavität mit dem pulverförmigen Arzneimittel herstellerseitig in dem Lager des Gehäuses angeordnet. Die Blisterkavität zur Aufnahme des Arzneimittels hat sich insofern bewährt, als sie einen wirksamen Schutz vor Umgebungseinflüssen darstellt.

Um das zu inhalierende Arzneimittel und den Inhalator vor Umgebungseinflüssen zu schützen, ist der Inhalator mit einer luftdichten Umverpackung, insbesondere einem Folienbehälter, versehen. Eine derartige Umverpackung ist handelsüblich. Alternativ oder zusätzlich sind das Mundstück und/oder die Luftansaugöffnung mit einer abnehmbaren Kappe dicht verschlossen. Aufgrund dieser Maßnahmen ist der Innenraum des Inhalators mit dem Arzneimittel mit einem minimalen Verpackungsaufwand vor insbesondere das Arzneimittel schädigenden Einflüssen, wie beispielsweise Feuchtigkeit, geschützt.

In weiterer Ausgestaltung der Erfindung ist das Arzneimittel in der Blisterkavität, in Strömungsrichtung gesehen, vor der Einheit zum Dispergieren des pulverförmigen Arzneimittels gelagert und gelangt durch eine zentrale Bohrung der Einheit in den Inhalationskanal des Mundstücks, wobei die Einheit mindestens eine radiale Zuströmöffnung aufweist, die mit einer in den Inhalationskanal mündenden Strömungsbohrung verbunden ist. Beim Inhalieren strömt Luft durch die eine Öffnung in der Blisterkavität und nimmt das Arzneimittel durch die andere Öffnung in der Blisterkavität in die zentrale Bohrung der Einheit zum Dispergieren des Arzneimittels mit. Die durch die radiale Zuströmöffnung in die Einheit strömende Luft sorgt für eine Verwirbelung des Arzneimittels, womit eine Feinverteilung der Pulverpartikel des Arzneimittels in der Strömungsbohrung einhergeht. Im weiteren Strömungsverlauf gelangen die Pulverpartikel in den Inhalationskanal, der im Gehäuseoberteil des Inhalators ausgebildet ist und von dort in die Lunge des Benutzers des Inhalators. Um eine hohe Förderrate des Arzneimittels sicherzustellen, ist die zentrale Bohrung der Einheit fluchtend zu einem der Anstechelemente ausgerichtet.

Zur Erzeugung einer hohen Strömungsgeschwindigkeit ist die zentrale Bohrung von einem luftdurchströmten Ringraum umgeben, in den die Zuströmöffnung mündet, wobei die Außenwahdung der Strömungsbohrung eine Innenwandung des Ringraums überragt.

Um eine gerichtete Strömung beim Inhalieren zu erzeugen, die einen möglichst hohen Anteil kleiner Pulverteilchen erzeugt, sind zwei diametral gegenüberliegende Zuströmöffnungen vorgesehen, deren Breite geringfügig größer als der Durchmesser der Innenwandung bemessen ist. In einer alternativen Ausgestaltung sind mindestens zwei zueinander versetzte Zuströmöffnungen vorgesehen, die in miteinander verbundene Strömungskanäle auf gegenüberliegenden Seiten der Einheit münden. Damit werden gegenläufig zyklonartige Strömungen erzielt. Vorzugsweise weist die Innenwandung axiale Durchbrüche zur Strömungsverbindung der Bohrung mit der Strömungsbohrung auf. Es ist auch möglich, dass die Einheit in ihrer Strömungsbohrung eine Prallplatte für das Arzneimittel aufweist. Alternativ ist die Prallplatte an einem in die Einheit eingesetzten Zentrierring befestigt. Sämtliche Maßnahmen zur Leitung der Luftströmung beim Inhalieren dienen der Erzeugung eines möglichst feinen Pulvers durch ein Zertrümmern größerer Arzneimittelpartikel.

Es versteht sich, dass die vorstehend genannten und nachstehend noch zu erläuternden Merkmale nicht nur in der jeweils angegebenen Kombination, sondern auch in anderen Kombinationen verwendbar sind. Der Rahmen der Erfindung ist nur durch die Ansprüche definiert.

Die Erfindung wird im Folgenden anhand mehrerer Ausführungsbeispiele unter Bezugnahme auf die zugehörigen Zeichnungen näher erläutert. Es zeigt:

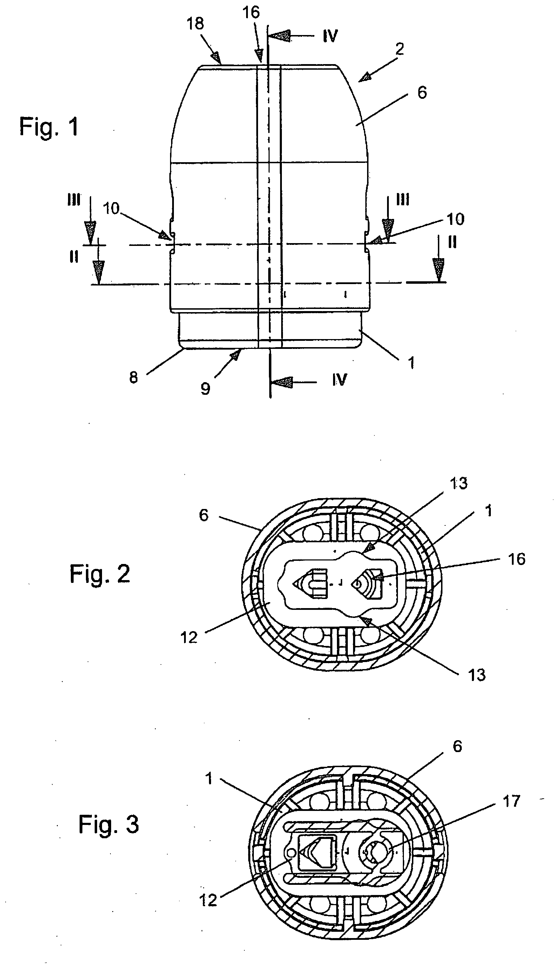

- Fig.1: eine Vorderansicht eines erfindungsgemäßen Inhalators,
- Fig.2: eine Schnittdarstellung des Inhalators nach Fig. 1 gemäß der Linie II-II,
- Fig.3: eine Schnittdarstellung des Inhalators nach Fig. 1 gemäß der Linie III-III,

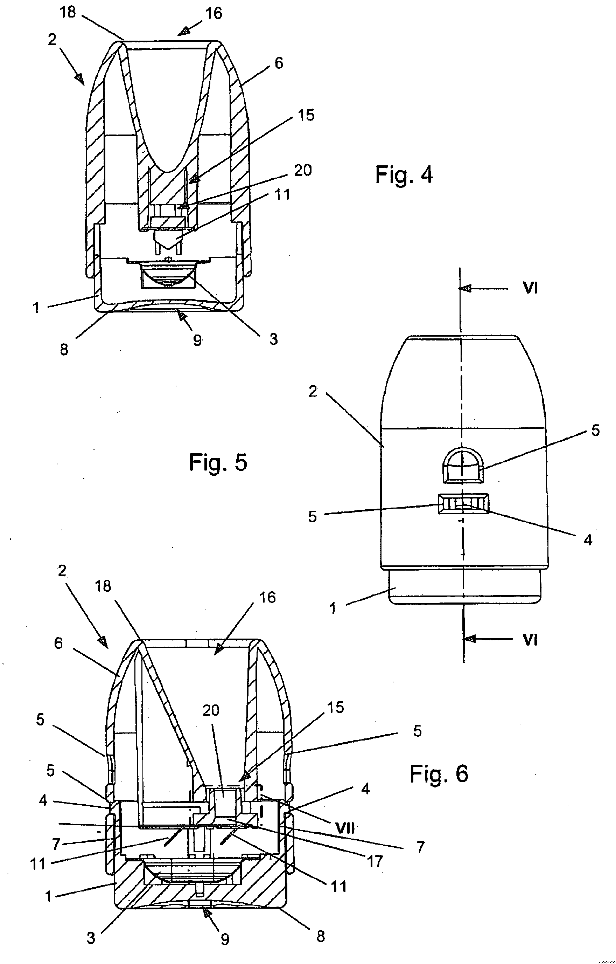

- Fig.4: eine Schnittdarstellung des Inhalators nach Fig. 1 gemäß der Linie IV-IV,
- Fig.5: eine Seitenansicht des Inhalators nach Fig. 1,
- Fig.6: eine Schnittdarstellung des Inhalators nach Fig. 5 gemäß der Linie VI-VI,
- Fig.7: eine vergrößerte Seitenansicht der Darstellung der Einzelheit VII nach Fig. 6,
- Fig.8: eine Schnittdarstellung der Einzelheit nach Fig. 7 gemäß der Linie VIII-VIII,
- Fig.9: eine Schnittdarstellung der Einzelheit nach Fig. 7 gemäß der Linie IX-IX,
- Fig.10: eine Schnittdarstellung der Einzelheit nach Fig. 7 gemäß der Linie X-X,
- Fig.11: eine vergrößerte Seitenansicht der Darstellung der Einzelheit VII nach Fig. 6 in alternativer Ausgestaltung,
- Fig.12: eine Schnittdarstellung der Einzelheit nach Fig. 11 gemäß der Linie XII-XII,
- Fig.13: eine Schnittdarstellung der Einzelheit nach Fig. 11 gemäß der Linie XIII-XIII,
- Fig.14: eine Schnittdarstellung der Einzelheit nach Fig. 11 gemäß der Linie XIV-XIV,
- Fig.15: eine vergrößerte Seitenansicht der Darstellung der Einzelheit VII nach Fig. 6 in zweiter alternativer Ausgestaltung,
- Fig.16: eine Schnittdarstellung der Einzelheit nach Fig. 15 gemäß der Linie XVI-XVI,
- Fig.17: eine Schnittdarstellung der Einzelheit nach Fig. 15 gemäß der Linie XVII-XVII,
- Fig.18: eine Schnittdarstellung der Einzelheit nach Fig. 15 gemäß der Linie XVIII-XVIII,
- Fig.19: eine vergrößerte Seitenansicht der Darstellung der Einzelheit VII nach Fig. 6 in dritter alternativer Ausgestaltung,
- Fig.20: eine Schnittdarstellung der Einzelheit nach Fig. 19 gemäß der Linie XX-XX,
- Fig.21: eine Schnittdarstellung der Einzelheit nach Fig. 19 gemäß der Linie XXI-XXI,
- Fig.22: eine Schnittdarstellung der Einzelheit nach Fig. 19 gemäß der Linie XXII-XXII,
- Fig.23: eine vergrößerte Seitenansicht der Darstellung der Einzelheit VII nach Fig. 6 in vierter alternativer Ausgestaltung,
- Fig.24: eine Schnittdarstellung der Einzelheit nach Fig. 23 gemäß der Linie XXIV-XXIV,
- Fig.25: eine Schnittdarstellung der Einzelheit nach Fig. 23 gemäß der Linie XXV-XXV und
- Fig.26: eine Schnittdarstellung der Einzelheit nach Fig. 25 gemäß der Linie XXVI-XXVI.

Der Inhalator dient zur Verabreichung eines pulverförmigen Arzneimittels aus einer Blisterkavität, die auf einem einem Gehäuseunterteil 1 eines Gehäuses 2 ortsfest zugeordneten, im Querschnitt wannenförmigen Lager 3 herstellerseitig angeordnet ist. Das Gehäuseunterteil 1 ist mit zwei gegenüberliegenden, an freien Enden von Rastarmen 7 angeordneten Clipsnasen 4 versehen, die in dazu korrespondierende Clipsöffnungen 5 in unterschiedlichen Ebenen eines als Mundstück ausgebildeten Gehäuseoberteils 6 eingelassen sind. Im Weiteren weist das Gehäuseunterteil 1 in seinem Boden 8 Lufteintrittsöffnungen 9 auf, durch die Luft aus der Umgebung beim Inhalieren in das Innere des Gehäuses 2 strömt. Im Auslieferungszustand des Inhalators, in dem die Blisterkavität ungeöffnet in dem Gehäuse 2 aufgenommen ist, greifen die Clipsnasen 4 in die Clipsöffnungen 5, die zum Boden des Gehäuseunterteils 1 benachbart sind und bilden erste Clipsverbindungen 10, wobei in dieser Lage des Gehäuseunterteils 1 zu dem Gehäuseoberteil 6 Anstechelemente 11 zum Öffnen der Blisterkavität zu dieser beabstandet sind. Zum Öffnen der Blisterkavität wird das Gehäuseunterteil 1 relativ zu dem Gehäuseoberteil 6 verschoben, wobei die Clipsnasen 4 in die langlochförmigen Clipsöffnungen 5, die zum oberen Ende des Gehäuseoberteils 6 benachbart sind, eingreifen und zweite Clipsverbindungen bilden. Aufgrund der ersten Clipsverbindungen 10 und der zweiten Clipsverbindungen hat der Benutzer des Inhalators sowohl eine optische als auch eine taktile Rückmeldung über den Zustand der Blisterkavität. Um ein Verdrehen des Gehäuseoberteils 6 zu dem Gehäuseunterteil 1 zu vermeiden, weisen diese einen elliptischen Querschnitt auf, wobei sich das Gehäuseoberteil 6 in Richtung seines oberen Endes verjüngt, um eine ergonomisch günstige Anlagefläche für die Lippen eines Benutzers des Inhalators zu bilden.

Die Anstechelemente 11 sind zueinander beabstandet in einer Platte 12 aus Metall freigestanzt und in einem spitzen Winkel derart zu der Platte 24 abgewinkelt, dass sie in Richtung des Lagers 3 für die Blisterkavität weisen. Um eine relativ große Öffnung in einer Deckfolie der Blisterkavität bei einer verhältnismäßig geringen Krafteinwirkung zu erzeugen, weist jedes Anstechelement eine dreieckförmige Spitze auf. Die Platte 12 ist mit Zentrierbohrungen 13 versehen, in die Zentrierbolzen 14 einer Einheit 15 zum Dispergieren des pulverförmigen Arzneimittels eingreifen, die nach einer Druckbeaufschlagung im erwärmten Zustand die Platte 12 dauerhaft haltern. Die Platte 12 ist derart ausgerichtet, dass ein Anstechelement 11 im Bereich eines Inhalationskanals 16 des Gehäuseoberteils 6 und ein Anstechelement 11 seitlich dazu versetzt angeordnet ist. Die separat zu fertigende Einheit 15 ist fest in den Inhalationskanal 16 des Mundstücks eingesetzt.

Das in der Blisterkavität, in Strömungsrichtung gesehen, vor der Einheit 15 zum Dispergieren des pulverförmigen Arzneimittels gelagerte Arzneimittel gelangt beim Inhalieren durch eine zentrale Bohrung 17 der Einheit 15 in den sich in Richtung einer Luftaustrittsöffnung 18 erweiternden Inhalationskanal 16 des Mundstücks, wobei sich die zentrale Bohrung 17 oberhalb des dem Inhalationskanal 16 des Gehäuseoberteils 6 zugeordneten Anstechelements 11 befindet.

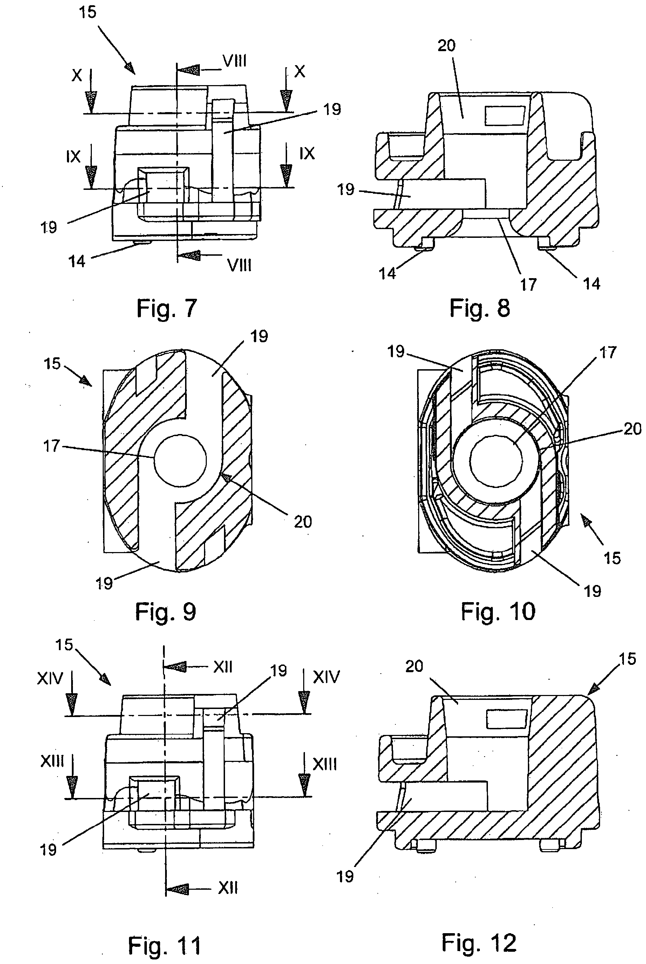

Die Einheit 15 weist nach den Fig. 7 bis 10 auf jeder Seite zwei jeweils außermittig angeordnete sowie zueinander versetzte Zuströmöffnungen 19 für Luft aus der Umgebung auf, die tangential in eine mit dem Inhalationskanal 16 verbundene Strömungsbohrung 20 münden, die quasi eine Verlängerung der zentralen Bohrung 17 darstellt. Aufgrund der Anordnung der Zuströmöffnungen 19 entstehen beim Ansaugen zyklonartige Wirbelströmungen in der Strömungsbohrung 20, die gemeinsam mit der durch die zentrale Bohrung 17 strömenden Luft, die mit dem Arzneimittel beladen ist, für eine feine Verteilung der Pulverpartikel des Arzneimittels sorgen.

Auch bei der Einheit 15 nach den Fig. 11 bis 14 sind auf jeder Seite die beiden jeweils außermittig in der Einheit 15 angeordneten sowie zueinander versetzten Zuströmöffnungen 19 für Luft aus der Umgebung vorgesehen, die tangential in die mit dem Inhalationskanal 16 verbundene Strömungsbohrung 20 münden, wobei die zentrale Bohrung 17 als kreisabschnittförmiger Durchbruch 21 mit abgerundeten Ecken 22 ausgeführt ist.

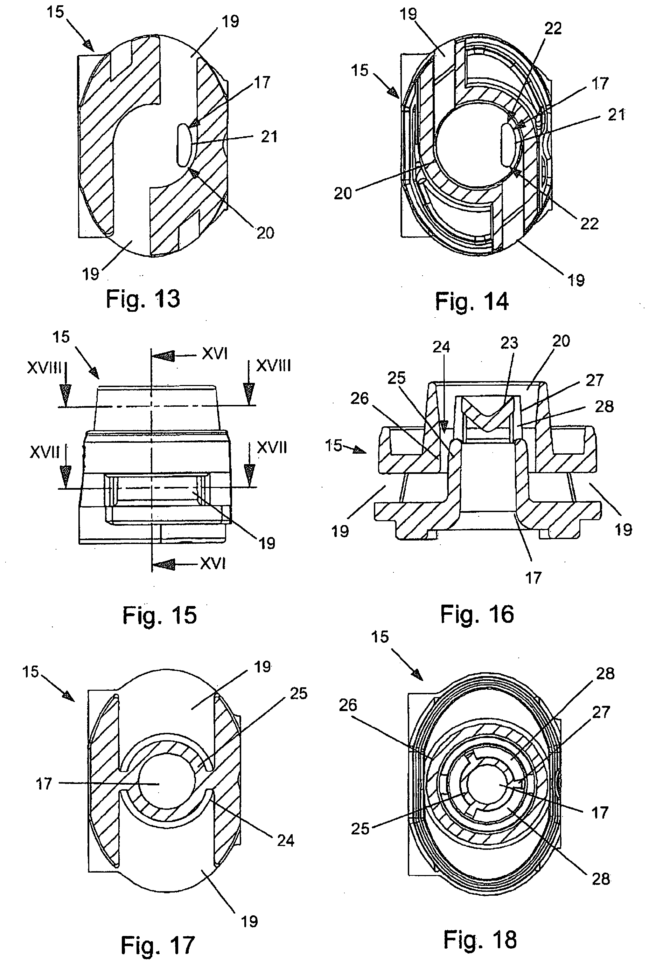

Die Einheit 15 gemäß den Fig. 15 bis 18 ist mit einer Prallplatte 23 ausgestattet, die sich konisch über die zentrale Bohrung 17 koaxial in der Strömungsbohrung 20 erstreckt, wobei die Spitze 24 der konischen Prallplatte 23 in Richtung des Lagers 3 für die Blisterkavität mit dem Arzneimittel weist und die Prallplatte 23 einen kleineren Durchmesser aufweist als die Strömungsbohrung 20, so dass zwischen der zentralen Bohrung 17 und der Strömungsbohrung 20 ein Ringraum 24 mit einer Innenwandung 25 gebildet ist, durch den angesaugte Luft strömt, wobei eine Außenwandung 26 der Strömungsbohrung 20 die Innenwandung 25 des Ringraums 24 und die Prallplatte 23 überragt. Die Prallplatte 23 ist über Stege 27 mit der Innenwandung 25 verbunden, wonach umfangsseitige Durchbrüche 28 zur Strömungsverbindung der zentralen Bohrung 17 mit der Strömungsbohrung 20 gebildet sind. Zur weiteren Verwirbelung der mit dem Arzneimittel beladenen Luftströmung sind die beiden Zuströmöffnungen 19 diametral gegenüberliegend in der Einheit 15 angeordnet und weisen eine Breite auf, die geringfügig größer als der Durchmesser der Innenwandung 25 bemessen ist.

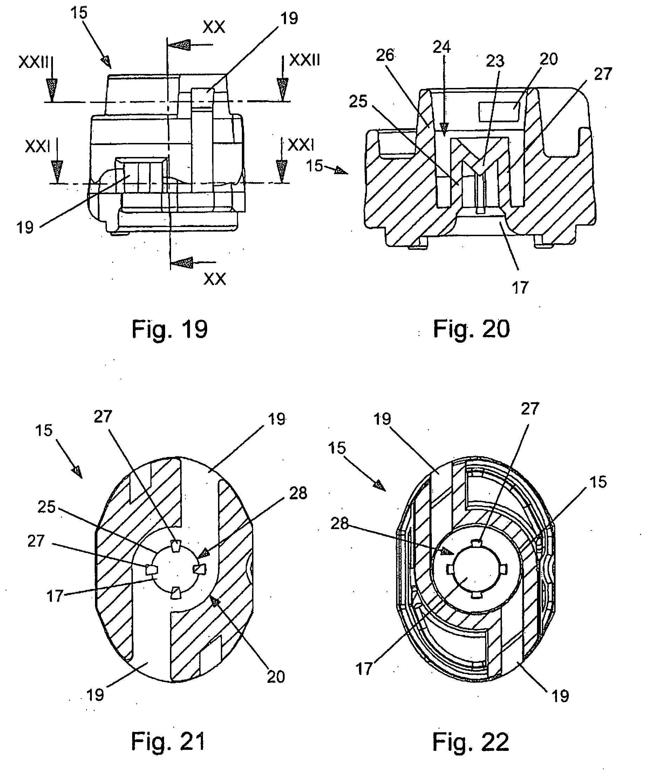

Bei der Einheit 15 entsprechend den Fig. 19 bis 22 sind in jeder Seite die beiden jeweils außermittig in der Einheit 15 angeordneten sowie zueinander versetzten Zuströmöffnungen 19 für Luft aus der Umgebung eingelassen, die tangential zunächst in den Ringraum 24 und anschließend senkrecht dazu in die Strömungsbohrung 20 münden. Die die zentrale Bohrung 17 überspannende Prallplatte 23 ist, wie zuvor erläutert, konisch ausgebildet und koaxial sowohl zu der zentralen Bohrung 17 als auch zu der Strömungsbohrung 20 ausgerichtet.

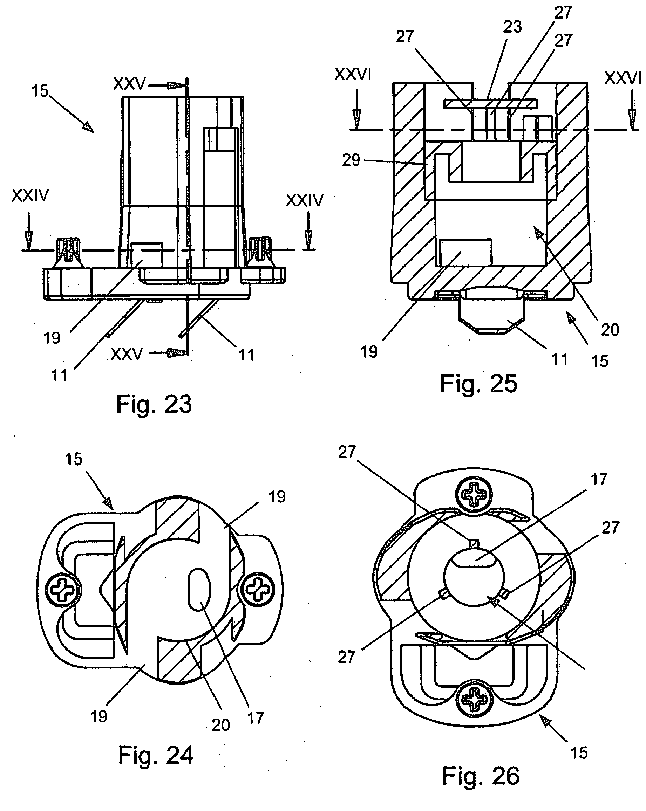

Bei der Einheit 15 nach den Fig. 23 bis 26 sind in jeder Seite die beiden jeweils außermittig in der Einheit 15 angeordneten sowie zueinander versetzten Zuströmöffnungen 19 für Luft aus der Umgebung eingelassen, die tangential in die Strömungsbohrung 20 münden, die durch die Prallplatte 23 überspannt ist. Die ebene Prallplatte 23 ist über die Stege 27 mit einem in die Einheit 15 eingesetzten Zentrierring 29 verbunden. Der Außendurchmesser der Prallplatte 23 ist größer als der Durchmesser der Strömungsbohrung 20 im Bereich des Zentrierrings 29 bemessen.

Es wurde ein Standardtest zum Vergleich eines Inhalationsgerätes mit Anstechelement mit einem Inhalationsgerät mit Anstechelement und Prallplatte durchgeführt. Als Testsubstanz diente eine Standartformulierung mit BIBW 2948. Der Versuch diente der Untersuchung der Pulverausbringung. Die folgenden Testresultate wurden gefunden.

Dosierung: 30mg der 25% Versuchsformulierung (7,5 mg Wirkstoff)

| Versuch | 1 | 2 |
|---|---|---|
| Inhalationsgerät | Standard mit Anstechelement ohne Prallplatte | Standard mit Anstechelement und mit Prallplatte |
| FPD _{(4kPa)} < µm / mg (Vk / %) | 1,5 (23,7) | 1,9 (8,9) |
| MMAD / µm (GSD) | 4,2 (1,9) | 3,9 (1,9) |
| FPD _{(1kPa)}/FPD _{(4kPa)} | 0.27 | 0.56 |
| | | |
| FPF _{(4kPa)}/% | 20.5 | 24,7 |

## Patentansprüche

1. Inhalator zur Verabreichung eines pulverförmigen Arzneimittels in Form einer inhalationsfähigen Substanz, Substanzformulierung oder -mischung aus einer mit Anstechelementen (11) zu öffnenden Blisterkavität, die in einem Gehäuseunterteil (1) eines Gehäuses (2) gelagert ist, das aus einem als Mundstück ausgebildeten Gehäuseoberteil (6) mit einem Inhalationskanal (16) und dem eine Lufteintrittsöffnung (9) aufweisenden Gehäuseunterteil (1) zusammengesetzt ist, wobei der Inhalationskanal (16) des Gehäuseoberteils (6) eine Einheit (15) zum Dispergieren des pulverförmigen Arzneimittels aufweist, die mit den Anstechelementen (11) verbunden ist, wobei das Gehäuseoberteil (6) zum Öffnen der Blisterkavität relativ zu dem Gehäuseunterteil (1) verlagerbar ist,
wobei das Arzneimittel in der Blisterkavität, in Strömungsrichtung gesehen, vor der Einheit (15) zum Dispergieren des pulverförmigen Arzneimittels gelagert ist,
so dass beim Inhalieren Arzneimittel durch eine zentrale Bohrung (17) der Einheit (15) in den Inhalationskanal (16) des Mundstücks gelangt, wobei die Einheit (15) mindestens
zwei zueinander versetzte Zuströmöffnungen (19) hat, die tangential mit einer in den Inhalationskanal (16) mündenden Strömungsbohrung (20) verbunden sind, so dass zyklonartige Wirbelströmungen in der Strömungsbohrung (20) entstehen;
und wobei die Einheit (15) separat gefertigt und fest in den Inhalationskanal (16) des Mundstücks eingesetzt ist.

2. Inhalator nach Anspruch 1, **dadurch gekennzeichnet, dass** die Anstechelemente (11) in einer durch mindestens eine erste Clipsverbindung (10) zwischen dem Gehäuseoberteil (1) und dem Gehäuseunterteil (6) arretierten Lage beabstandet zu der Blisterkavität ausgerichtet sind und in einer durch mindestens eine zweite Clipsverbindung definierten Lage in die geöffnete Blisterkavität hineinragen.

3. Inhalator nach Anspruch 1 oder 2, **dadurch gekennzeichnet, dass** jedes Anstechelement (11) eine dreieckförmige Spitze aufweist, wobei ein Anstechelement (11) im Bereich des Inhalationskanals (16) und ein Anstechelement (11) seitlich dazu versetzt angeordnet ist.

4. Inhalator nach einem der Ansprüche 1 bis 3, **dadurch gekennzeichnet, dass** die Anstechelemente (11) aus einem Kunststoff gefertigt sind.

5. Inhalator nach einem der Ansprüche 1 bis 4, **dadurch gekennzeichnet, dass** die Anstechelemente (11) einstückig mit der Einheit (15) zum Dispergieren gefertigt sind.

6. Inhalator nach einem der Ansprüche 1 bis 3, **dadurch gekennzeichnet, dass** die Anstechelemente (11) aus Metall bestehen.

7. Inhalator nach Anspruch 6, **dadurch gekennzeichnet, dass** die Anstechelemente (11) in einer Platte (12) aus Metall freigestanzt und in einem spitzen Winkel der-art zu der Platte (12) abgewinkelt sind, dass sie in Richtung der Blisterkavität weisen.

8. Inhalator nach Anspruch 7, **dadurch gekennzeichnet, dass** die Platte (12) form- und oder kraftschlüssig mit der Einheit (15) zum Dispergieren verbunden ist.

9. Inhalator nach Anspruch 1 oder 2, **dadurch gekennzeichnet, dass** das Gehäuseun-terteil (1) zwei gegenüberliegende Clipsnasen (4) aufweist und in das Gehäuse-oberteil (6) dazu korrespondierende Clipsöffnungen (5) in unterschiedlichen Ebenen eingelassen sind.

10. Inhalator nach Anspruch 9, **dadurch gekennzeichnet, dass** die Clipsnasen (4) an freien Enden von gegenüberliegenden Rastarmen (7) angeordnet sind.

11. Inhalator nach Anspruch 9, **dadurch gekennzeichnet, dass** die Clipsöffnungen (5) in der Ebene, in der die Blisterkavität geöffnet ist, langlochförmig gestaltet sind.

12. Inhalator nach Anspruch 1 oder 2, **dadurch gekennzeichnet, dass** das Gehäuseoberteil (6) relativ zu dem Gehäuseunterteil (1) verschiebbar gelagert ist.

13. Inhalator nach Anspruch 1, 2 oder 12, **dadurch gekennzeichnet, dass** zwischen dem Gehäuseoberteil (6) und dem Gehäuseunterteil (1) eine Verdrehsicherung angeordnet ist.

14. Inhalator nach einem der Ansprüche 1 bis 13, **dadurch gekennzeichnet, dass** das Gehäuseoberteil (6) einen zylindrischen oder elliptischen Querschnitt aufweist, in dem das im Querschnitt zylindrische oder elliptische Gehäuseunterteil (1) ge-lagert ist und in Richtung einer Luftaustrittsöffnung (18) konisch verläuft, wobei die Lufteintrittsöffnung (9) an der freien Stirnseite des Gehäuseunterteils (1) ausgebildet ist.

15. Inhalator nach einem der Ansprüche 1 bis 13, **dadurch gekennzeichnet, dass** das Gehäuseoberteil und das Gehäuseunterteil jeweils im Querschnitt wannenförmig ausgebildet sind, wobei das Gehäuseunterteil in dem Gehäuseoberteil gelagert ist.

16. Inhalator nach Anspruch 1, **dadurch gekennzeichnet, dass** die Blisterkavität mit dem pulverförmigen Arzneimittel herstellerseitig in dem Lager (3) des Gehäuses (2) angeordnet ist.

17. Inhalator nach einem der Ansprüche 1 bis 16, **dadurch gekennzeichnet, dass** der Inhalator mit einer luftdichten Umverpackung, insbesondere einem Folienbehälter, versehen ist.

18. Inhalator nach Anspruch 1, **dadurch gekennzeichnet, dass** die zentrale Bohrung (17) der Einheit (15) fluchtend zu einem der Anstechelemente (11) ausgerichtet ist.

19. Inhalator nach Anspruch 1, **dadurch gekennzeichnet, dass** die zentrale Bohrung (17) von einem luftdurchströmten Ringraum (24) umgeben ist, in den die Zuströmöffnung (19) mündet, wobei die Aussenwandung (26) der Strömungsbohrung (20) eine Innenwandung (25) des Ringraums (24) überragt.

20. Inhalator nach Anspruch 1 oder 19, **dadurch gekennzeichnet, dass** zwei diametral gegenüberliegende Zuströmöffnungen (19) vorgesehen sind, deren Breite ge-ringfügig grösser als der Durchmesser der Innenwandung (25) bemessen ist.

21. Inhalator nach Anspruch 1 oder 19, **dadurch gekennzeichnet, dass** mindestens zwei zueinander versetzte Zuströmöffnungen (19) vorgesehen sind, die in miteinander verbundene Strömungskanäle auf gegenüberliegenden Seiten der Einheit (15) münden.

22. Inhalator nach einem der Ansprüche 19 bis 21, **dadurch gekennzeichnet, dass** die Innenwandung (25) axiale Durchbrüche (21) zur Strömungsverbindung der Bohrung (17) mit der Strömungsbohrung (20) aufweist.

23. Inhalator nach einem der Ansprüche 1 oder 19 bis 22, **dadurch gekennzeichnet, dass** die Einheit (15) in ihrer Strömungsbohrung (20) eine Prallplatte (23) für das Arzneimittel aufweist.

24. Inhalator nach Anspruch 23, **dadurch gekennzeichnet, dass** die Prallplatte (23) an einem in die Einheit eingesetzten Zentrierring (29) befestigt ist.

25. Inhalator nach einem der Ansprüche 1 bis 24, **gekennzeichnet durch** seine Einmalverwendbarkeit.

## Claims

1. Inhaler for administration of a pulverulent medicament in the form of an inhalable substance, substance formulation or substance mixture from a blister cavity which is to be opened by piercing elements (11) and is mounted in a lower housing part (1) of a housing (2) which is composed of an upper housing part (6), which is in the form of a mouthpiece and has an inhalation channel (16), and the lower housing part (1), which has an air inlet opening (9), wherein the inhalation channel (16) of the upper housing part (6) has a unit (15) for dispersing the pulverulent medicament, which unit is connected to the piercing elements (11), the upper housing part (6) being displaceable relative to the lower housing part (1) in order to open the blister cavity wherein the medicament is stored in the blister cavity, viewed in the direction of flow, upstream of the unit (15) for dispersing the pulverulent medicament, such that on inhalation, medicament passes through a central bore (17) of the unit (15) into the inhalation channel (16) of the mouthpiece, the unit (15) having at least two mutually offset tangential inflow openings (19) which communicate with a flow bore (20) that leads into the inhalation channel (16) so that a cyclone-like turbulent flow is produced in the flow bore (20) and wherein the unit (15) is manufactured separately from the inhalation channel (16) of the mouthpiece and then fixedly inserted into it.

2. Inhaler according to claim 1, **characterised in that** the piercing elements (11) are disposed at a distance from the blister cavity in a position locked by at least one first clip connection (10) between the upper housing part (1) and the lower housing part (6) and, in a position defined by at least one second clip connection, they project into the opened blister cavity.

3. Inhaler according to claim 1 or 2, **characterised in that** each piercing element (11) has a triangular tip, one piercing element (11) being arranged in the region of the inhalation channel (16) and one piercing element (11) being arranged offset laterally thereto.

4. Inhaler according to any one of claims 1 to 3, **characterised in that** the piercing elements (11) are made of a plastics material.

5. Inhaler according to any one of claims 1 to 4, **characterised in that** the piercing elements (11) are produced in one piece with the unit (15) for dispersion.

6. Inhaler according to any one of claims 1 to 3, **characterised in that** the piercing elements (11) are made of metal.

7. Inhaler according to claim 6, **characterised in that** the piercing elements (11) are punched out of a plate (12) made of metal and are bent at an acute angle to the plate (12) so that they point in the direction towards the blister cavity.

8. Inhaler according to claim 7, **characterised in that** the plate (12) is connected in a positive-locking and/or friction-locked manner to the unit (15) for dispersion.

9. Inhaler according to claim 1 or 2, **characterised in that** the lower housing part (1) has two opposing clip projections (4), and corresponding clip openings (5) are let into the upper housing part (6) in different planes.

10. Inhaler according to claim 9, **characterised in that** the clip projections (4) are arranged at free ends of opposing locking arms (7).

11. Inhaler according to claim 9, **characterised in that** the clip openings (5) in the plane in which the blister cavity is opened are in the form of slots.

12. Inhaler according to claim 1 or 2, **characterised in that** the upper housing part (6) is mounted to be displaceable relative to the lower housing part (1).

13. Inhaler according to claim 1, 2 or 12, **characterised in that** an anti-twist means is arranged between the upper housing part (6) and the lower housing part (1).

14. Inhaler according to any one of claims 1 to 13, **characterised in that** the upper housing part (6) has a cylindrical or elliptical cross-section in which the lower housing part (1), which is cylindrical or elliptical in cross-section, is mounted and extends conically in the direction towards an air outlet opening (18), the air inlet opening (9) being formed on the free end face of the lower housing part (1).

15. Inhaler according to any one of claims 1 to 13, **characterised in that** the upper housing part and the lower housing part are each dish-shaped in cross- section, the lower housing part being mounted in the upper housing part.

16. Inhaler according to claim 1, **characterised in that** the blister cavity containing the pulverulent medicament is arranged by the manufacturer in the bearing (3) of the housing (2).

17. Inhaler according to any one of claims 1 to 16, **characterised in that** the inhaler is provided with an air-tight outer packaging, in particular a film container.

18. Inhaler according to claim 1, **characterised in that** the central bore (17) of the unit (15) is disposed in alignment with one of the piercing elements (11).

19. Inhaler according to claim 1, **characterised in that** the central bore (17) is surrounded by an annular space (24) through which air flows and into which the inflow opening (19) leads, the outer wall (26) of the flow bore (20) overlapping an inner wall (25) of the annular space (24).

20. Inhaler according to claim 1 or 19, **characterised in that** two diametrally opposite inflow openings (19) are provided, the width of which is slightly greater than the diameter of the inner wall (25).

21. Inhaler according to claim 1 or 19, **characterised in that** there are provided at least two mutually offset inflow openings (19) which lead into flow channels on opposite sides of the unit (15) which communicate with one another.

22. Inhaler according to any one of claims 19 to 21, **characterised in that** the inner wall (25) has axial apertures (21) for the flow connection of the bore (17) with the flow bore (20).

23. Inhaler according to any one of claims 1 or 19 to 22, **characterised in that** the unit (15) has in its flow bore (20) a deflector plate (23) for the medicament.

24. Inhaler according to claim 23, **characterised in that** the deflector plate (23) is fastened to a centering ring (29) inserted into the unit.

25. Inhaler according to any one of claims 1 to 24, **characterised in that** it is for single use.

## Revendications

1. Inhalateur pour l'administration d'un médicament pulvérulent sous la forme d'une substance, formulation de substance ou mélange de substances à inhaler, constitué d'une cavité coque à ouvrir avec des éléments perforateurs (11), qui est stockée dans une partie inférieure de boîtier (1) d'un boîtier (2) qui est constitué d'une partie supérieure de boîtier (6) réalisée sous forme d'embouchure avec un conduit d'inhalation (16) et de la partie inférieure de boîtier (1) présentant une ouverture d'entrée d'air (9), le conduit d'inhalation (16) de la partie supérieure de boîtier (6) présentant une unité (15) pour disperser le médicament pulvérulent, laquelle est raccordée aux éléments perforateurs (11), la partie supérieure de boîtier (6) pouvant être déplacée par rapport à la partie inférieure de boîtier (1) pour ouvrir la cavité coque,
le médicament étant stocké dans la cavité coque, vu dans la direction d'écoulement, avant l'unité (15) pour disperser le médicament pulvérulent,
de telle sorte que lors de l'inhalation, le médicament parvienne à travers un orifice central (17) de l'unité (15) dans le conduit d'inhalation (16) de l'embouchure, l'unité (15) ayant au moins deux ouvertures d'afflux (19) décalées l'une par rapport à l'autre, qui sont connectées tangentiellement à un orifice d'écoulement (20) débouchant dans le conduit d'inhalation (16) de telle sorte que des écoulements tourbillonnaires de type cyclone se produisent dans l'orifice d'écoulement (20) ;
et l'unité (15) étant fabriquée séparément et étant insérée fixement dans le conduit d'inhalation (16) de l'embouchure.

2. Inhalateur selon la revendication 1, **caractérisé en ce que** les éléments perforateurs (11) sont orientés dans une position à distance de la cavité coque, bloquée par au moins une première connexion par enclipsage (10) entre la partie supérieure de boîtier (1) et la partie inférieure de boîtier (6), et pénètrent dans la cavité coque ouverte dans une position définie par au moins une deuxième connexion par enclipsage.

3. Inhalateur selon la revendication 1 ou 2, **caractérisé en ce que** chaque élément perforateur (11) présente une pointe triangulaire, un élément perforateur (11) étant disposé dans la région du conduit d'inhalation (16) et un élément perforateur (11) étant disposé de manière décalée latéralement par rapport à celui-ci.

4. Inhalateur selon l'une quelconque des revendications 1 à 3, **caractérisé en ce que** les éléments perforateurs (11) sont fabriqués en une matière plastique.

5. Inhalateur selon l'une quelconque des revendications 1 à 4, **caractérisé en ce que** les éléments perforateurs (11) sont fabriqués d'une seule pièce avec l'unité (15) de dispersion.

6. Inhalateur selon l'une quelconque des revendications 1 à 3, **caractérisé en ce que** les éléments perforateurs (11) se composent de métal.

7. Inhalateur selon la revendication 6, **caractérisé en ce que** les éléments perforateurs (11) sont découpés par estampage dans une plaque (12) en métal et sont coudés à angle aigu par rapport à la plaque (12) de telle sorte qu'ils soient tournés dans la direction de la cavité coque.

8. Inhalateur selon la revendication 7, **caractérisé en ce que** la plaque (12) est connectée par engagement par correspondance géométrique et/ou par force à l'unité (15) de dispersion.

9. Inhalateur selon la revendication 1 ou 2, **caractérisé en ce que** la partie inférieure de boîtier (1) présente deux ergots d'enclipsage opposés (4) et des ouvertures d'enclipsage (5) correspondant à ceux-ci sont réalisées dans la partie supérieure de boîtier (6) dans des plans différents.

10. Inhalateur selon la revendication 9, **caractérisé en ce que** les ergots d'enclipsage (4) sont disposés au niveau d'extrémités libres de bras d'encliquetage opposés (7).

11. Inhalateur selon la revendication 9, **caractérisé en ce que** les ouvertures d'enclipsage (5) sont configurées sous forme de trous oblongs dans le plan dans lequel la cavité coque est ouverte.

12. Inhalateur selon la revendication 1 ou 2, **caractérisé en ce que** la partie supérieure de boîtier (6) est supportée de manière déplaçable par rapport à la partie inférieure de boîtier (1).

13. Inhalateur selon la revendication 1, 2 ou 12, **caractérisé en ce qu'**entre la partie supérieure de boîtier (6) et la partie inférieure de boîtier (1) est disposée une fixation antirotation.

14. Inhalateur selon l'une quelconque des revendications 1 à 13, **caractérisé en ce que** la partie supérieure de boîtier (6) présente une section transversale cylindrique ou elliptique dans laquelle la partie inférieure de boîtier (1) de section transversale cylindrique ou elliptique est supportée et s'étend sous forme conique dans la direction d'une ouverture de sortie d'air (18), l'ouverture d'entrée d'air (9) étant réalisée au niveau du côté frontal libre de la partie inférieure de boîtier (1).

15. Inhalateur selon l'une quelconque des revendications 1 à 13, **caractérisé en ce que** la partie supérieure de boîtier et la partie inférieure de boîtier sont chacune réalisées sous forme de cuvette en section transversale, la partie inférieure de boîtier étant supportée dans la partie supérieure de boîtier.

16. Inhalateur selon la revendication 1, **caractérisé en ce que** la cavité coque est disposée avec le médicament pulvérulent par le fabricant dans le support (3) du boîtier (2).

17. Inhalateur selon l'une quelconque des revendications 1 à 16, **caractérisé en ce que** l'inhalateur est pourvu d'un emballage étanche à l'air, en particulier d'un récipient en feuille.

18. Inhalateur selon la revendication 1, **caractérisé en ce que** l'orifice central (17) de l'unité (15) est orienté de manière alignée avec l'un des éléments perforateurs (11).

19. Inhalateur selon la revendication 1, **caractérisé en ce que** l'orifice central (17) est entouré par un espace annulaire (24) parcouru par de l'air dans lequel débouche l'ouverture d'afflux (19), la paroi extérieure (26) de l'orifice d'écoulement (20) dépassant au-delà d'une paroi intérieure (25) de l'espace annulaire (24).

20. Inhalateur selon la revendication 1 ou 19, **caractérisé en ce que** deux ouvertures d'afflux diamétralement opposées (19) sont prévues, dont la largeur est dimensionnée de manière légèrement supérieure au diamètre de la paroi intérieure (25).

21. Inhalateur selon la revendication 1 ou 19, **caractérisé en ce qu'**au moins deux ouvertures d'afflux décalées l'une par rapport à l'autre (19) sont prévues, lesquelles débouchent dans des conduits d'écoulement connectés l'un à l'autre sur des côtés opposés de l'unité (15).

22. Inhalateur selon l'une quelconque des revendications 19 à 21, **caractérisé en ce que** la paroi intérieure (25) présente des orifices axiaux (21) pour la liaison fluidique de l'orifice (17) avec l'orifice d'écoulement (20).

23. Inhalateur selon l'une quelconque des revendications 1 ou 19 à 22, **caractérisé en ce que** l'unité (15) présente, dans son orifice d'écoulement (20), une plaque d'impact (23) pour le médicament.

24. Inhalateur selon la revendication 23, **caractérisé en ce que** la plaque d'impact (23) est fixée au niveau d'une bague de centrage (29) insérée dans l'unité.

25. Inhalateur selon l'une quelconque des revendications 1 à 24, **caractérisé par le fait qu'**il peut être utilisé une fois.
